# EUROPEAN PATENT APPLICATION

(11) **EP 0 955 061 A1**
(43) Date of publication of application: **10.11.1999**
(21) Application number: 99301120.4
(22) Date of filing: 16.02.1999
(51) Int. Cl.: A61K 39/40, A61K 39/42, A61K 35/74, A61K 31/07, A61K 31/355, A61K 31/59

(54) **Oral product for the prevention and therapy of porcine gastroenteric infections**

(30) Priority: 20.03.1998 CZ 85998
(71) Applicant: Medipharm CZ, s.r.o., 693 01 Hustopece u Brna (CZ)
(72) Inventor: Mican, Petr, 693 01 Hustopece u Brna (CZ); Stepánek, Jan, 592 56 Zvole nad Perstynem (CZ)
(74) Representative: Lawrence, John Gordon

(57) **Abstract**

Oral product for the prevention and therapy of porcine gastrointestoinal infections containing at least one specific antibody to porcine rotavirus, porcine coronavirus, enteropathogenic and enterotoxigenic bacterial strains of *Escherichia coli, Clostridium sp., Salmonella sp.* and protozoan strains of *Isospora sp.* and *Cryptosporidium sp,* obtained from egg yolks of immunized hens. Further, the product contains at least one strain of live stabilized cultures of lactacidogenic bacteria. Technology of production consisting of separate submersive culture of selected individual strains of lactacidogenic bacterial species *Enterococcus faecium, Lactobacillus casei* and, if appropriate, *Lactobacillus plantarum,* followed by the separation of the bacterial cells from the medium, their preservation by freeze-drying, and in blending of individual species or a combination thereof with the antibodies and the excipient of the product.

## Description

### Field of invention

The invention pertains to an oral product for the prevention and therapy of infectious diseases of the gastrointestinal tract of swine.

### Background

The elementary prerequisite of economical production of piglets and slaughter pigs is to maintain them in a good state of health throughout the rearing period. The industrial character of pig production poses many problems and makes it a demanding task for herd owners. Housing of large populations in spatially limited premises represents an unnatural and serious challenge to the immune system of the animals, resulting often in reduced resistance to infections. At the same time, it provides favourable conditions for survival and stepwise increase of virulence of many pathogens, particularly facultatively pathogenic agents, persisting permanently and in increasing concentrations in the barns.

In epizootiological terms, such state can be characterized as artificial creation of conditions suitable for the development of numerous infections affecting above all the gastrointestinal and respiratory tracts and resulting eventually in massive outbreaks of diarrhoeic or respiratory diseases. Infections of the gastrointestinal tract are of particular importance, because they affect all age groups of swine, particularly piglets and weaners. Irrespective of the etiologic agent, the clinical picture of gastrointestinal infections is rather uniform including diarrhoea of various severities and types, dehydration, dysorexia or anorexia, loss of body condition and decrease of weight gains. Death rate in piglets and weaners reaches 20% or more and can become an important economic factor also in older pigs. The etiology of gastrointestinal infections is rather diverse, the agents including viruses, bacteria and, to a lesser extent, protozoa, or combinations thereof. The most significant of them are porcine rotavirus, porcine coronavirus (causative agent of transmissive gastroenteritis - TGE), porcine coronavirus (causative agent of porcine epidemic diarrhoea - PED), enterotoxigenic and enteropathogenic strains of *Escherichia coli* (ETEC and EDEC, at least four serotypes), *Clostridium sp.* (particularly *Clostridium perfringens* Type A), *Salmonella sp., Serpulina hyodysenteriae* (causative agent of porcine dysentery), *Serpulina pilosicoli* (causative agent of porcine intestinal spirochaetosis), *Lawsonia intracellularis* (causative agent of porcine proliferative enteropathy), and *Isospora suis* and *Cryptosporidium* (protozoan species accompanying and complicating gastrointestinal infections induced by the above agents).

Of particular importance to the herd owner are porcine rotavirus, ETEC and *Clostridium sp.,* inducing endemic and recurrent infections in sucking and weaned piglets, and *Salmonella spp, Serpulina hyodysetneriae* and porcine coronavirus (PED) responsible for endemic and recurrent infections in weaners and growing pigs.

Factors limiting the efficacy of prophylactic measures include multifactorial etiology, facultative pathogenicity of the causative agents, and local character of the infections. Owing to the frequent occurrence of gastrointestinal infections, measures at their control are an essential part of herd management, however.

The current measures include the control of environmental contamination, vaccination and treatment with antibiotics.

General measures of infection control, such as current environmental sanation, are inevitable, but their effect is only insufficient and transient owing to the potential of enteropathogenic microorganisms to survive in the organisms of normal hosts.

The efficacy of oral or parenteral treatment with antibiotics is also insufficient, even when large doses are administered to all animals at risk. Antibiotics are quite ineffective in the case of virus infections. Oral administration may affect the propagation of bacteria and thus contribute to a favourable course of the infection. However, this effect is limited to the period of administration and is often accompanied by unwanted temporary immunosuppression, development of resistant bacterial strains and suppression of intestinal microflora essential for normal digestive processes

A certain preventive effect can be expected from the administration of probiotics that suppress the propagation of enteropathogenic or enterotoxigenic bacteria on a principle of competitive inhibition, and enhance the activity of the immune system, particularly the local immunologically active structures of the intestinal tract.

The result of vaccination is also unsatisfactory, because only antibodies present in the intestine, either produced by immunocompetent intestinal structures or received orally, are immunologically active . Parenteral vaccination does not induce local immunity and the development of oral vaccines has not yet reached the stage of commercial production. A partial effect has been achieved only in vaccines intended for enhancing the protection of newborn piglets. The vaccines are administered to pregnant sows with the aim to increase the concentration of antibodies in colostrum and milk. The antibodies are received by piglets throughout the sucking period, and protect the intestinal mucosa by neutralizing enteropathogenic microorganisms in the intestinal lumen and inducing passive local immunity stopping the development of the infection. A drawback of the vaccination of pregnant sows consists in the often incomplete antigenic spectrum of the appropriate biologicals and the fact that antibodies are present in mammary gland secretions only for a limited period and that their concentration is liable to a number of factors, such as mastitis, agalactia, or inappropriate interventions by attendants.

Generally, efforts to induce passive local immunity of the gastrointestinal tract by continuous oral administration of specific blood serum antibodies (hyperimmune or convalescent blood sera) has proved to be impractical. The major reason thereof was a failure of attempts to produce the necessary amount of antibodies with the required range of specificity at acceptable costs. Another drawback of such local immunization is the rapid denaturation of blood serum antibodies in the gastrointestinal tract. There exists evidence enough, based on the current knowledge of pathogenesis of gastrointestinal infections, that parenteral administration of antibodies is ineffective.

### Principle of invention

The oral product for the prevention and therapy of porcine gastrointestinal infections eliminates, the above drawbacks to a considerable extent. The preventive and therapeutic activity of the product as per invention consists in the combination of two prophylactic and therapeutic principles, i.e. specific passive immunotherapy with the antibodies and non-specific prophylactic activity of stabilized cultures of lactacidogenic bacteria - probiotics. The specific component of the product are antibodies to one or more enteropathogenic agents, displaying their activity in the gastrointestinal lumen by neutralization the surface antigens (pathogenicity factors) of bacteria and inhibition of their adhesion to the gastrointestinal mucosa and permanent growth on its surface, or penetration of viruses into and their replication in epithelial cells. Thus, the development of the infection is hampered and its extent and severity are limited. The other active component is a stabilized culture of lactacidogenic bacteria *Enterococcus sp.* capable of competitive inhibition of adherence and propagation of enterotoxigenic and enteropathogenic bacteria in the gastrointestinal tract of piglets and pigs. The cultures not only limit the risk of bacterial sepsis, but also contribute to the maintenance of eubiosis in the intestinal lumen of piglets, including those affected by viral infections, and thus enhance the local effect of the specific antibodies. These two synergetic components of the product can be completed with further supportive substances, such as vitamins, and suitable excipients ensuring a standard content of the active components, homogeneity and long-term stability of the product, and facilitating its administration.
The specific antibodies are obtained from eggs of hens immunized with antigens of porcine rotavirus (such as strain CAPM V-334), porcine coronavirus (such as strains CAPM V-66, CAPM V-126, CAPM V-474), porcine enteropathogenic and enterotoxigenic strains of *E. coli* (such as CAPM 5051 through 5057), *Salmonella sp.* strains (such as CAPM 5938, CAPM 5939, CAPM 5445), *Clostridium sp.* strains (such as CAPM 5744), *Serpulina sp.* strains (such as CAPM 6033, CAPM 6163) and, as appropriate, protozoan species *Isospora* and *Cryptosporidium.* The raw material, in this case liquid eggs, obtained from the immunized donors is homogenized and preserved by spray, or fluid, or freeze-drying. The latter method should be preferred because the biological potential of the input material is fully preserved. The resulting product is a white or light yellow loose powder. The minimal acceptable neutralization titre of antibodies to rotavirus and coronavirus in the dried colostrum or egg yolks is 16 for 100 TCID₅₀ (50% Tissue Culture Infectious Dose) per 0.05 g dried product. The agglutination or precipitation titre of antibodies to bacterial and protozoan agents must reach at least 20 and 4, respectively, for 10⁸ inactivated bacterial cells per 1.00 g dried product.
The other active component of the product are stabilized live cultures of lactacidogenic bacteria *Enterococcus spp.* and/or *Lactobacillus spp.,* such as *Enterococcus faecium,* strain M 74 - CCM 6226, or *Lactobacillus casei,* such as strain CCM 3775, or *Lactobacillus plantarum,* such as strain CCM 3769. The probiotic component of the product is obtained by submersive pulsative culture of the appropriate strains. The bacterial biomass resulting from the exponential growth phase is separated from the medium, supplemented with a cryoprotective agent and stabilized by freeze-drying. The concentration of live lactoacidogenic bacterial cells shall reach at least 50 x 10⁹ CFU (Colony Forming Units) per 1 g of the dry probiotic concentrate.

To increase the metabolic rate and enhance the resistance of the organism to infection, it is recommendable to supplement the product with A, D₂ and E vitamin concentrates in dry, oil or water-soluble forms. The minimal daily doses should be 1000 IU vitamin A, 100 IU vitamin D₂ and 5 mg vitamin E per animal.

The product is intended exclusively for oral administration to piglets, weaners and older pigs at various stages of the postnatal development and therefore a paste formula and a powder formula have been devised.

The paste is intended above all for oral administration to newborn and sucking piglets reared conventionally in individual pens. The product is packaged in special plastic applicators allowing accurate and reliable dosage without any risk of aspiration.

The water-soluble powder formula (pulvis, premix) allows both individual dosage with a measuring jar, and group dosage of the product mixed into water or combined feeds throughout the rearing period (weaners, finishing pigs). The powder formula is also suitable for mixing into rehydration solutions and other products intended for symptomatic therapy of diarrhoea of calves.

The major excipient components of the paste are colloidal silicone hydrolysate and distilled monoglycerides that, along with quality edible oil, form a consistence with a molecular mesh ensuring a uniform dispersion of the active components. The personnel involved in paste processing must adhere to rules of aseptic work in all operations. Dried skim milk or dried whey combined with saccharose, glucose, sorbitol and lactose are used as excipients in the powder formula.

The major benefit of the oral product for the prevention and therapy of porcine gastrointestinal infections as per invention consists in the combined action of two separate preventive and therapeutic processes, i.e. passive immunotherapy with specific antibodies and inhibitory activity of the probiotic bacterial cultures. The neutralizing activity of the specific antibodies in the intestinal lumen of piglets and pigs is enhanced by the competitive inhibitory effect of lactacidogenic bacteria against a number of enteropathogenic agents. Several indisputable benefits for practical application result also from the fact that the antibodies inducing passive immunity in piglets and pigs are prepared from egg yolks of immunized hens. This procedure allows the manufacturer to obtain large amounts of specific antibodies at favourable production costs. Owing to the high specificity, the antibodies can either be used separately as a monovalent semi-finished article, or composed into mixtures with polyvalent activities corresponding to the spectrum of agents responsible for the rise and development of the gastrointestinal disease.

Alternatively, the donor hens can be immunized with a combination of antigens to obtain yolk mass containing a defined range of antibodies corresponding to the specific antigenic structures of the causative agents.

Compared with blood serum antibodies, the antibodies present in egg yolk are more resistant to low pH and proteolytic enzymes and remain active in the gastrointestinal tract.

Another advantage resulting from the use of eggs as the source of antibodies is the composition of the product consisting exclusively of biological materials corresponding to common protein components used in the nutrition of swine.

The probiotic component of the product allows a purposeful choice and use of various competitively inhibiting, adherence, growth and metabolic activities of individual strains of lactacidogenic bacteria, such as *Enterococcus faecium,* strain M 74 CCM 6226), *Lactobacillus plantarum* (CCM 3769), or *Lactobacillus casei* (CCM 3775). Either a single bacterial strain or a combination of two or three species can be used. All the bacterial species are non-pathogenic and represent a significant favourable symbiotic component of the intestinal microbial population of well being animals, such as piglets or pigs. Therefore, no hazards of inappropriate use or overdosage exist.

### Examples of application of the invention

### Example 1

Paste is the most suitable administration formula for newborn piglets, because it allows safe and accurate dosage without any risk of aspiration by the treated animal. The paste is manufactured by blending individual active components, i.e. specific antibodies, as are those to rotavirus, coronavirus and/or enteropathogenic bacteria, with a concentrate of lactacidogenic bacteria. The antibodies contained in lyophilized or otherwise dried egg yolks collected from immunized hens are thoroughly blended with freeze-dried concentrated cultures of *Enterococcus faecium, Lactobacillus casei* and/or *Lactobacillus plantarum* and a mixture of vitamin A, D₃ and E concentrates. In the next step, this mixture of the active components is stepwise added into a homogenous mixture of excipient substances including quality edible oil, such as groundnut oil, colloidal siloid-hydrolysate, such as CABOSIL, and a mixture of distilled monoglycerides, such as MYVEROL. The complete mixture is then processed in an vacuum homogenizer and finally filled into plastic applicators containing, for instance, 40 or 80 ml of the product. The normal oral dose is 1 to 5 ml. Animals at acute risk can be treated with double doses.

**Table 1 :**

| Basic composition of the paste formula of the product | |
|---|---|
| **Carrier components** | **percentage (w/w)** |
| Quality edible oil (such as groundnut oil) | 40 - 60 |
| Colloidal siloid (such as CABOSIL) | 3 - 7 |
| Distilled monoglycerides | 1 - 3 |

| **Active components** | |
|---|---|
| Dried (freeze-dried) egg yolks of immunized hens | 18 - 40 |
| Lactacidogenic bacteria concentrate CFU 200 x 10⁹/g *(Enterococcus faecium* M 74, *Lactobacillus plantarum)* | 4 - 5 |

| **Vitamin supplement** | |
|---|---|
| Mixture of vitamin A, D3 and E concentrates | 1 - 2 |

### Example 2

The water-soluble powder formula is manufactured in standard blenders (such as Nautimax) equipped for both horizontal and vertical blending of the components. The active components, including dried (freeze-dried) egg yolks of immunized hens and concentrated freeze-dried cultures of lactacidogenic bacteria, are first homogenized in a small blender and then transferred into a large blender containing a smaller amount of the excipient. After thorough blending, the remaining volume of the excipient is added and the blendng is finished. The product as per invention is packed in alufan bags, or plastic containers (such as alufan bags containing 500 g product, or plastic containers or bags containing up to 25 kg product).

The dosage of the product as per invention is individual within the range of 1.5 to 3 g per piglet per day, or 500 to 250 g per 1 ton dry milk-based combined feed, or complete combined feed.

**Table 2:**

| Basic components of the powder (pulvis/premix) formula of the product as per invention | |
|---|---|
| **Carrier components** | **percentage (w/w)** |
| Dried instant milk or dried whey | 16 - 40 |
| Glucose, lactose, sorbitol, saccharose (starch) | 16 - 45 |

| **Active components** | |
|---|---|
| Dried (freeze-dried egg yolks of immunized animals | 2 - 60 |
| Lactacidogenic bacteria concentrate CFU: 5 x 10⁹/1 g *Enterococcus faecium* | 10 - 15 |

## Claims

1. Oral product for the prevention and therapy of gastrointestinal infections of swine, characterized by the content of at least one specific antibody to porcine rotavirus, porcine coronavirus, enterotoxigenic and enteropathogenic strains of bacterial species *Escherichia coli, Clostridium sp., Salmonella sp., Serpulina sp.* and protozoan species *Isospora sp.* and *Cryptosporidium sp.,* obtained from egg yolks of immunized hens,.

2. Product as per Claim 1, characterized by the presence of at least one strain of a live stabilized culture of lactacidogenic bacteria .

3. Product as per Claim 1, characterized by the amount of antiviral antibodies of at least 16/100 TCID₅₀ and/or the amount of antibacterial antibodies corresponding to the agglutination titre 20 or precipitation titre 4 per 10⁸ inactivated bacteria per 1 g product.

4. Product as per Claim 2, characterized by the fact that the live stabilized cultures of the lactacidogenic bacteria are strains of *Enterococcus faecium, Lactobacillus casei* and/or *Lactobacillus plantarum.*

5. Product as per Claim 4 characterized by the concentration of stabilized live cultures of lactacidogenic bacteria within the range of 5 x 10³ to 50 x 10⁹ live cells per 1 g product.

6. Product as per Claim 1 characterized by the presence of vitamins and excipients.

7. Product as per Claim 6, characterized by the amount of vitamins corresponding to at least 1000 IU for vitamin A, at least 100 IU for vitamin D₃, and at least 5 mg for vitamin E per piglet per day.

8. Product as per Claim 6 characterized by the presence of excipimets including 40 to 60% (w/w) edible oil, 3 to 7% (w/w) colloidal siloids, and 1 to 3% (w/w) distilled monoglycerides.

9. Products as per Claim 6 characterized by the presence in the excipient of 16 to 45% (w/w) glucose, and/or saccharose, and/or lactose, and/or sorbitol, and of 16 to 40% (w/w) dried milk and/or dried whey.

10. Technology of the production of the product as per Claims 1 though 9, characterized by separate production of the probiotic component of the product by submersive culture of individual selected lactacidogenic bacteria *Enterococcus faecium, Lactobacillus casei* and *Lactobacillus plantarum,* that are, after the culture is finished, separated from the medium, preserved by freeze-drying and eventually blended as individual components or a combination thereof with the antibodies the excipient.

11. Technology of the production as per Claim 10, characterized by immunization of hens with separate antigens of porcine rotavirus, porcine coronavirus, enteropathogenic and enterotoxigenic strains of *Escherichia coli, Clostridium sp., Salmonella sp., Serpulina sp.,* and, if appropriate, strains of *Isospora sp.,* and *Cryptosporidium sp.,* or an appropriate combination of the antigens, whereupon yolks or liquid eggs obtained from the immunized hens are preserved by fluid, spray, of freeze-drying.

12. Technology of the production as per Claim 11, characterized by the facts that the porcine rotavirus is the strain CAPM V-334, the porcine coronaviruses are the strains CAPM V-66, CAPM V-126, and CAPM V-474, the *Escherichia coli* strains are CAPM 5051, CAPM 5052, CAPM 5053, CAPM 5054, CAPM 5055, CAPM 5056, and CAPM 5057, the strain of *Clostridium sp.* is CAPM 5744, the strains of *Salmonella sp.* are CAPM 5938, CAPM 5939, and CAPM 5445, and the strains of *Serpulina sp.* are CAPM 6063 and CAPM 6163.
